# EUROPEAN PATENT SPECIFICATION

(11) **EP 2 560 952 B1**
(45) Date of publication and mention of the grant of the patent: **01.10.2014**
(21) Application number: 11714566.4
(22) Date of filing: 18.04.2011
(51) Int. Cl.: C07D 207/34, C07D 405/06

(54) **PRODUCTION OF ATORVASTATIN LOW IN LACTONE IMPURITIES**
Herstellung von Atorvastatin mit geringen Lactonverunreinigungen
Production d'atorvastatine à faible teneur en impuretés de lactone

(30) Priority: 06.09.2010 EP 10175441; 19.04.2010 EP 10160281
(43) Date of publication of application: 27.02.2013
(73) Proprietor: DSM Sinochem Pharmaceuticals Netherlands B.V., 2613 AX Delft (NL)
(72) Inventor: LANGE, DE, Ben, NL-6151 GE Munstergeleen (NL)
(74) Representative: De Vroom, Erik
(86) International application number: PCT/EP2011/056094
(87) International publication number: WO 2011/131601

(56) References cited:
- WO-A1-2006/011155
- WO-A2-2006/048893
- WO-A2-2009/019561

## Description

### Field of the invention

The present invention relates to a method for the production of atorvastatin.

### Background of the invention

Atorvastatin ([*R*-(*R**,*R**)]-2-(4-fluorophenyl)-β,δ-dihydroxy-5-(1-methylethyl)-3-phenyl-4-[(phenylamino)carbonyl]-1*H*-pyrrole-1-heptanoic acid hemi calcium salt, (**2**)) is a pharmaceutical ingredient useful as an inhibitor of the enzyme 3-hydroxy-3-methylglutaryl-coenzyme A reductase (HMG-CoA reductase) and thus useful as a hypolipidemic and hypocholesterolemic agent.

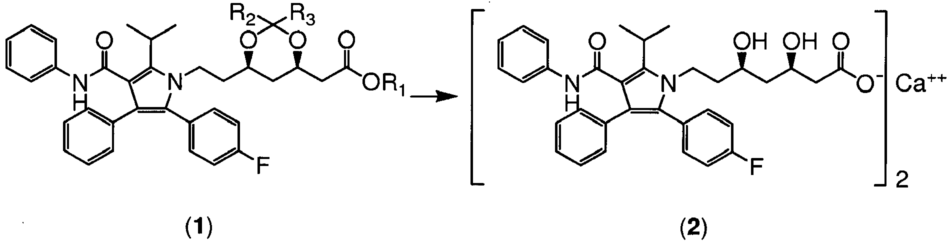

The preparation of atorvastatin is well known and the most common approach is based upon protection/deprotection of the carboxylic acid function as a *tert*-butyl ester ((**1**); R₁ = -C(CH₃)₃) and of the diol functionality as an acetonide ((**1**); R₂ = R₃ = -CH₃). This has been described in WO 2008/129562 and in the many references cited therein.

As with any active pharmaceutical intermediate, also for atorvastatin strict control of impurities in the end product is of pivotal importance. The general reaction sequence described above utilizing *tert*-butyl ester protection of the carboxylic acid function has been investigated intensively. The unique chemical nature of the *tert*-butyl ester function has leads to a unique spectrum of impurities, both in terms of chemical structure as well as in terms of relative amounts. Various solutions have been given for reducing the amounts of impurities in the documents mentioned above. However, all these solutions apply to *tert-*butyl ester protection strategies.

Recently, another type of protection has been disclosed in WO 2009/019561, namely the use of the *iso*-propyl group for carboxylic acid protection ((**1**); R₁ = -CH(CH₃)₂). The differences in chemical reactivity of this group as compared to the *tert-*butyl ester function under the prior art deprotection conditions leads to differences in impurity profiles calling for different solutions.

WO2006011155 discloses:
A method of producing Atorvastatin hemi calcium salt from a tert-butyl ester precursor comprising the following steps:
   1. treating a formula (2) precursor solution with HCl,
   2. treating the mixture with NaOH,
   3. washing the mixture obtained, continuing with the aq. phase.
   4. adjusting pH of aq. layer to 7.5-8.5,
   5. treating the washed mixture with the calcium salt Ca(OAc)2.

WO2006011155 also discloses a claim to a general process of producing Atorvastatin hemi calcium salt

WO2006048893 discloses a process analogous to that of WO2006011155

### Detailed description of the invention

In a first aspect of the invention, the production of atorvastatin hemi calcium salt of formula (**2**) from a compound of general formula (**1**) wherein R₁ = -CH(CH₃)₂ and R₂ and R₃ are independently chosen from the list consisting of ethyl, methyl and propyl or wherein R₂ and R₃ form a cyclopentylidene or cyclohexylidene ring, is achieved by the steps of: 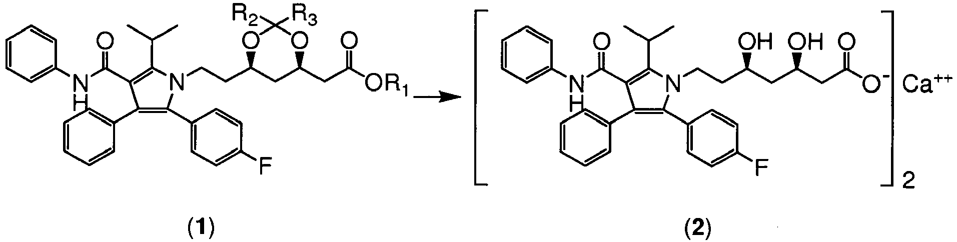
(a) Treating a solution of said compound of general formula (**1**) in a first solvent with an acid;
(b) Treating the mixture obtained in step (a) with an alkali metal hydroxide and washing it with a second solvent;
(c) Treating the washed mixture obtained in step (b) with a calcium salt or with calcium hydroxide,
   characterized in that a second acid is added after step (b)
   or after step (c) until the pH of the mixture is
   between 8.6 and 9.0

Suitable first solvents include acetonitrile, alcohols, cyclic ethers such as dioxane and tetrahydrofurane and ketones such as acetone and mixtures thereof. The first solvent may be an alcohol such as ethanol, *iso*-propanol, methanol and propanol. The acid used in step (a) may be an inorganic acid such as hydrobromic acid, hydrochloric acid, nitric acid, phosphoric acid or sulfuric acid. The amount of alkali metal hydroxide added in step (b) is such that the pH of the mixture is raised to a value between 11 and 13, preferably between 11.5 and 12.5. Suitable alkali metal hydroxides are lithium hydroxide, potassium hydroxide and sodium hydroxide. The calcium salt used in step (c) may be calcium acetate. Seed crystals of atorvastatin hemi calcium salt crystal form I may be added prior to the addition of the calcium salt in order to facilitate crystallization.

Atorvastatin hemi calcium salt precipitates after step (d) and can be isolated by filtration, centrifugation or other techniques known to the skilled person. Optionally the product thus obtained is further purified by re-slurrying in aqueous environment, preferably in water followed by isolation and drying of the crystals.

In the course of the process described above impurity (3) is formed.

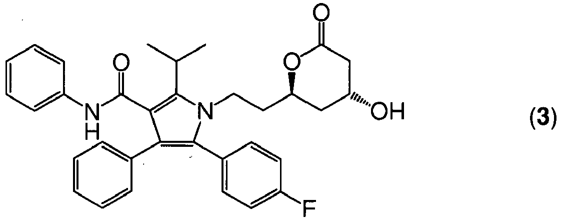

Unfortunately the amounts wherein this impurity is present in the final product may be as high as 0.1% which is unwanted in view of USP and/or European Pharmacopoeia (*Ph. Eur*.) requirements. Surprisingly it was found that the amount of impurity (**3**) could be lowered dramatically by lowering the pH of the mixture obtained after step (b) to a value not lower than 7.5, or not lower than 7.9, or not lower than 8.0. This can be achieved by adding an acid to the mixture obtained in step (b) or by adding an acid to the mixture obtained after step (c). In principle the commonly used organic and inorganic acids are suitable for the purpose of the present invention although in our hands acetic acid gave still more superior results. By applying the above measure atorvastatin hemi calcium salt crystal form I is obtained with levels of impurity (**3**) as low as from 0.0001 % to 0.06%.

It has been found that in specific cases it may be advantageous to perform a washing step with a second solvent in between process steps (b) and (c). Said second solvent may be an ether such as, for example, dibutyl ether, diethyl ether, methyl ether, methyl *tert*-butylether or an ester such as ethyl acetate or *iso*-propyl acetate or hydrocarbons such as toluene or petroleum ether.

Also described herein is a composition comprising atorvastatin hemi calcium salt and from 0.0001% to 0.06% by weight of a compound of general formula (**3**).

### EXAMPLES

### Example 1

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (75 kg, 117 mol) was added to 1125 L of methanol and stirred at 35-37°C until clarity. After cooling to 25-27°C aqueous HCl (made from 19 kg concentrated HCl and 64 L of water) was added and the reaction was stirred for 2 h at 25-27°C. The mixture was concentrated under vacuum in 3.5 h at 20-22°C to about 50% of its original volume. Then 825 L of methanol was added and the mixture stirred for 45 min. HPLC analysis revealed the starting material to be less than 0.03%. Aqueous NaOH (made from 15 kg NaOH and 560 L of water) was added keeping the temperature below 30°C to give a pH of 12.2 and stirring was continued for 2 h. The reaction mixture was concentrated to about 900 L under vacuum at a temperature of 20-22°C in 5 h. Next, 750 L of water and 450 L of methyl-t-butyl ether were added and stirred for 15 minutes after which phases were separated. Methyl-*t* butyl ether (450 L) was added and stirred for 15 min. after which phases were separated. After heating the aqueous layer to 35-37°C, 7.5 kg active carbon was added followed by stirring for 30 min. The reaction mixture was filtered through a Hyflo bed and the carbon/hyflo bed washed with water/methanol (135 L water/15 L methanol). The resulting solution was concentrated under vacuum, followed by addition of 150 L of water and 37.5 L of methyl-*t*-butyl ether. The temperature was increased to 45-47°C and the pH adjusted to 8.6-8.8 with aqueous acetic acid (3L acetic acid in 60 L of water). After heating the reaction mixture until 47-50°C, 7.5 kg of atorvastatin calcium polymorph I seed was added, followed by addition in 1 h of a solution of 14.5 kg Ca-acetate in 375 L of water. The mixture was heated to 55-58°C and maintained at this temperature for 30 min. The slurry was then cooled to 40-45°C and stirred for 3 h. The solid was isolated by centrifugation and the obtained wet-cake re-slurried in 1125 L of water at 40-45 °C. After stirring for 1 h, the solid was isolated by centrifugation and dried under vacuum at 50-55°C. Weight 63.5 kg. If required, the material can be milled, blended and/or micronized. Impurity: Lactone (3): 0.05%.

### Comparative Example 2 (pH at 7.2)

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (90 kg, 141 mol) was added to 1350 L of methanol and stirred at 35-37°C until clarity followed by cooling to 25-27°C. Then aqueous HCl (made from 22.8 kg concentrated HCl and 77 L of water) was added. The reaction was stirred for 2 h at 25-27°C. The mixture was then concentrated under vacuum in about 16 h at 29-30°C to about 30% of its original volume. Then 825 L of methanol was added and the mixture was concentrated under vacuum in 5 h at 29-30 °C to ∼40% of its original volume. Methanol (900 L) was charged followed by 620 L of aqueous 0.6 N NaOH keeping the temperature below 30°C to give a pH of not less than 12 (actual temperature 26°C and pH 12.4) and stirring was continued for 2 h. The reaction mixture was concentrated (∼900 L was distilled) under vacuum in 8 h at 29-30°C. Next, 810 L of water and 540 L of methyl-t-butyl ether were added and stirred for 15 minutes after which phases were separated. The aqueous layer was heated to 35-37°C, 5.0 kg active carbon added and stirred for 30 min. The reaction mixture was filtered through a Hyflo bed and the carbon/Hyflo bed washed with water/methanol (80 L water/10 L methanol). To the solution, 22 L of ethylacetate was added and stirred for 1 h. The pH was initially 8.3 but went down to approximately 7.2 during the next process steps. After heating the reaction mixture until 47-50°C, 9.0 kg of atorvastatin calcium polymorph I seed was added, followed by addition in 1 h of a solution of 14.0 kg Ca-acetate in 270 L of water. The mixture was heated to 55-58 °C and maintained at this temperature for 30 minutes. The slurry was then cooled to 40-45°C and stirred for 3 h. The solid was isolated by centrifugation and the wet-cake re-slurried in 900 L of water at 40-45°C. After stirring for 1 h, the solid was isolated by centrifugation and dried under vacuum at 50-55°C. Weight 75.9 kg. If required, the material can be milled, blended and/or micronized. Impurity: Lactone (**3**): 0.17%

### Comparative Example 3 (pH 7.1 during extractions steps)

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (30 g, 31 mmol) was added to 450 mL of methanol and at 33-35°C until a clear solution was obtained followed by cooling to 26-28°C. Then 36 mL of 2.2 N aqueous HCl was added in 15 min and the resulting reaction mixture stirred for 2 h at 26-28°C, when HPLC analysis revealed the starting material to be less than 0.1%. To the mixture, 205 mL of 0.6 N aqueous NaOH was added in 1 h keeping the temperature below 30°C. The pH was 12.3. After stirring for 2h, the clear solution was concentrated under vacuum at 27-29°C until a slurry was obtained. Then 300 mL of water and 180 mL of methyl-t-butyl ether were added. The phases were separated. The aqueous layer was extracted with a mixture of ethylacetate/cyclohexane (240 mL ethylacetate and 240 mL cyclohexane, 50/50). The phases were separated. The pH of the aqueous phase was ∼7.1. Thereafter, the aqueous phase was treated with 3.0 g active carbon. After filtration of the carbon, the reaction mixture was heated until 45-50°C, 60 mL of H₂O was added and the pH adjusted to 8.5 with 0.6 N aqueous NaOH. Then, 3.0 g of Atorvastatin Calcium Polymorph I seed was added, followed by addition in 1 h of a solution of 5.5 g Ca-acetate in 150 mL of water. The mixture was heated to 55-58 °C and maintained at this temperature for 30 minutes. The slurry was cooled to 40-45°C and stirred for 3 h. The solid was isolated by filtration and the wet-cake re-slurried in 400 mL of water. The slurry was heated to 40ºC, stirred for 1 h and filtered. The white solid was dried at 50-55ºC. Weight 22.8 g. Impurities: Lactone (3): 0.18%.

| **Table: Summary of results** | | |
|---|---|---|
| Example | pH control | Lactone (**3**) |
| 1 | 8.6-8.8 | 0.05 |
| 2 | 8.3 → 7.2 | 0.17 |
| 3 | 7.1 → 8.5 | 0.18 |

## Claims

1. Method for the production of atorvastatin hemi calcium salt of formula (**2**) from a compound of general formula (**1**) wherein R₁ = -CH(CH₃)₂ and R₂ and R₃ are independently chosen from the list consisting of ethyl, methyl and propyl or wherein R₂ and R₃ form a cyclopentylidene or cyclohexylidene ring, comprising the steps of: 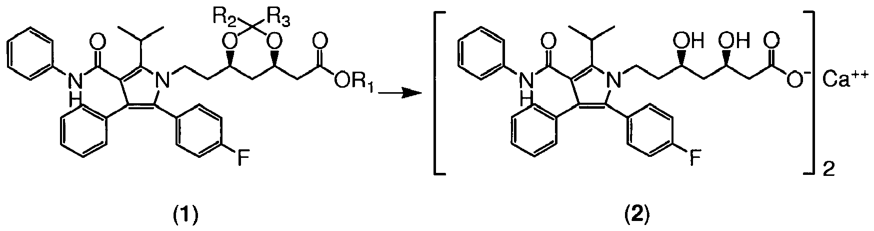
(a) treating a solution of said compound of general formula (1) in a first solvent with a first acid;
(b) treating the mixture obtained in step (a) with an alkali metal hydroxide and washing it with a second solvent,
(c) treating the washed mixture obtained in step (b) with a calcium salt or with calcium hydroxide,
**characterized in that** a second acid is added after step (b) or after step (c) until the pH of the mixture is between pH 8.6 and 9.0.

2. Method according to claim 1 wherein the pH after adding the second acid is between 8.6 and 8.8.

3. Method according to claim 1 or 2 wherein said second acid is acetic acid.

4. Method according to any one of claims 1 - 3 wherein both R₂ and R₃ are methyl.

5. Method according to any one of claims 1 to 4 wherein said first solvent is an alcohol.

6. Method according to any one of claims 1 to 5 wherein said first solvent is methanol.

7. Method according to any one of claims 1-6 wherein said second solvent is an ether.

## Patentansprüche

1. Verfahren zur Herstellung von Atorvastatin-Hemikalziumsalz der Formel (2) aus einer Verbindung der allgemeinen Formel (1), wobei R₁=-CH(CH₃)₂ und R₂ und R₃ unabhängig voneinander aus der aus Ethyl, Methyl und Propyl bestehenden Liste ausgewählt sind oder wobei R₂ und R₃ einen Cyclopentyliden- oder Cyclohexylidenring bilden, welches die folgenden Schritte umfasst: 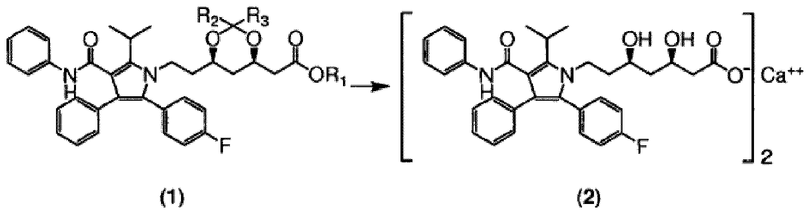
(a) Behandeln einer Lösung der Verbindung der allgemeinen Formel (1) in einem ersten Lösungsmittel mit einer ersten Säure,
(b) Behandeln der im Schritt (a) erhaltenen Mischung mit einem Alkalihydroxid und Waschen mit einem zweiten Lösungsmittel,
(c) Behandeln der im Schritt (b) erhaltenen gewaschenen Mischung mit einem Kalziumsalz oder mit Kalziumhydroxid,
**dadurch gekennzeichnet, dass** nach Schritt (b) oder nach Schritt (c) mit einer zweiten Säure versetzt wird, bis der pH-Wert der Mischung zwischen pH 8,6 und 9,0 liegt.

2. Verfahren nach Anspruch 1, wobei der pH-Wert nach der Zugabe der zweiten Säure zwischen 8,6 und 8,8 liegt.

3. Verfahren nach Anspruch 1 oder 2, wobei es sich bei der zweiten Säure um Essigsäure handelt.

4. Verfahren nach einem der Ansprüche 1-3, wobei sowohl R₂ als auch R₃ für Methyl stehen.

5. Verfahren nach einem der Ansprüche 1-4, wobei es sich bei dem ersten Lösungsmittel um einen Alkohol handelt.

6. Verfahren nach einem der Ansprüche 1-5, wobei es sich bei dem ersten Lösungsmittel um Methanol handelt.

7. Verfahren nach einem der Ansprüche 1-6, wobei es sich bei dem zweiten Lösungsmittel um einen Ether handelt.

## Revendications

1. Méthode de production de sel d'hémi-calcium d'atorvastatine de formule (2) à partir d'un composé de formule générale (1) dans laquelle R₁ = -CH(CH₃)₂ et R₂ et R₃ sont choisis indépendamment dans la liste constituée par éthyle, méthyle et propyle, ou dans laquelle R₂ et R₃ forment un cycle cyclopentylidène ou cyclohexylidène, comprenant les étapes consistant à : 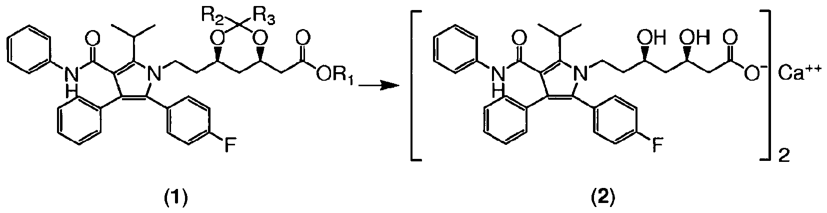
(a) traiter une solution dudit composé de formule générale (1) dans un premier solvant par un premier acide ;
(b) traiter le mélange obtenu dans l'étape (a) par un hydroxyde de métal alcalin et le laver par un deuxième solvant ;
(c) traiter le mélange lavé obtenu dans l'étape (b) par un sel de calcium ou par de l'hydroxyde de calcium ;
**caractérisée en ce qu'**un deuxième acide est ajouté après l'étape (b) ou après l'étape (c) jusqu'à ce que le pH du mélange soit compris entre pH 8,6 et 9,0.

2. Méthode selon la revendication 1, **caractérisée en ce que** le pH après l'addition du deuxième acide est compris entre 8,6 et 8,8.

3. Méthode selon la revendication 1 ou 2, **caractérisée en ce que** ledit deuxième acide est l'acide acétique.

4. Méthode selon l'une quelconque des revendications 1-3, **caractérisée en ce que** R₂ et R₃ sont tous deux méthyle.

5. Méthode selon l'une quelconque des revendications 1 à 4, **caractérisée en ce que** ledit premier solvant est un alcool.

6. Méthode selon l'une quelconque des revendications 1 à 5, **caractérisée en ce que** ledit premier solvant est le méthanol.

7. Méthode selon l'une quelconque des revendications 1-6, **caractérisée en ce que** ledit deuxième solvant est un éther.
